# EUROPÄISCHE PATENTANMELDUNG

(11) **EP 0 444 276 A2**
(43) Veröffentlichungstag der Anmeldung: **04.09.1991**
(21) Anmeldenummer: 90124293.3
(22) Anmeldetag: 15.12.1990
(51) Int. Cl.: C07D 249/08, C07D 303/04, C07C 33/50, A01N 43/653

(54) **1,2,4-Triazolyl-propanol-Derivate**

(30) Priorität: 28.02.1990 DE 4006223
(71) Anmelder: BAYER AG, 51368 Leverkusen (DE)
(72) Erfinder: Stroech, Klaus, Dr., W-5650 Solingen 19 (DE); Jautelat, Manfred, Dr., W-5093 Burscheid 1 (DE); Dehne, Heinz-Wilhelm, Dr., W-3061 Ahnsen (DE); Dutzmann, Stefan, Dr., W-4010 Hilden 1 (DE); Hänssler, Gerd, Dr., W-5090 Leverkusen 3 (DE)

(57) **Zusammenfassung**

Neue 1,2,4-Triazolyl-propanol-Derivate der Formel 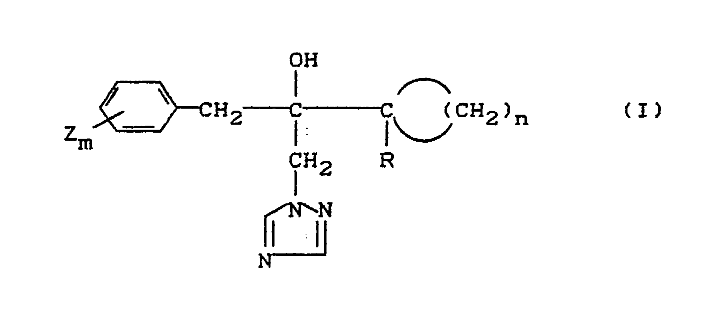
in welcher
- R: für Methyl oder Ethyl steht,
- Z: für Fluor, Chlor, Trifluormethyl, Trifluormethoxy, Trifluormethylthio, Methyl, gegebenenfalls durch Fluor oder Chlor substituiertes Phenyl oder gegebenenfalls durch Fluor oder Chlor substituiertes Phenoxy steht,
- m: für 0, 1, 2 oder 3 steht und
- n: für 4 oder 5 steht,
sowie deren Säureadditions-Salze und Metallsalz-Komplexe,
mehrere Verfahren zur Herstellung der neuen Stoffe und deren Verwendung als Fungizide.

Neue Zwischenprodukte, Verfahren zu deren Herstellung und deren Verwendung zur Synthese von 1,2,4-Triazolylpropanol-Derivaten der Formel (I).

## Beschreibung

Die vorliegende Anmeldung betrifft neue 1,2,4-Triazolylpropanol-Derivate, mehrere Verfahren zu deren Herstellung und deren Verwendung als Fungizide.

Es ist bereits bekannt, daß zahlreiche Azolylmethyl-cyclopropyl-Derivate fungizide Eigenschaften besitzen (vgl. EP-OS 0 297 345). So kann z.B. 1-(4-Chlorphenyl)-2-(1-methyl-cycloprop-1-yl)-3-(1,2,4-triazol-1-yl)-propan-2-ol zur Bekämpfung von Pilzen eingesetzt werden. Die Wirksamkeit dieses Stoffes ist gut; sie läßt allerdings bei niedrigen Aufwandmengen in manchen Fällen zu wünschen übrig.

Weiterhin ist bekannt, daß bestimmte durch Cycloalkyl substituierte Hydroxyethyl-azolyl-Derivate fungizide Eigenschaften besitzen (vgl. US-PS 4 551 469). Auch die Wirksamkeit dieser Stoffe ist aber nicht immer voll ausreichend.

Es wurden nun neue 1,2,4-Triazolyl-propanol-Derivate der Formel 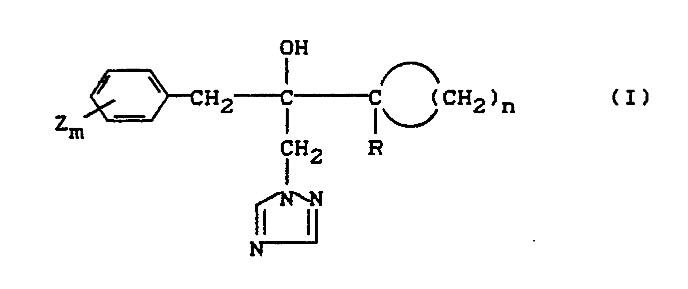
in welcher
- R: für Methyl oder Ethyl steht,
- Z: für Fluor, Chlor, Trifluormethyl, Trifluormethoxy, Trifluormethylthio, Methyl, gegebenenfalls durch Fluor oder Chlor substituiertes Phenyl oder gegebenenfalls durch Fluor oder Chlor substituiertes Phenoxy steht,
- m: für 0, 1, 2 oder 3 steht und
- n: für 4 oder 5 steht,

sowie deren Säureadditions-Salze und Metallsalz-Komplexe gefunden.

Weiterhin wurde gefunden, daß man 1,2,4-Triazolyl-propanol-Derivate der Formel (I) sowie deren Säureadditions-Salze und Metallsalz-Komplexe erhält, wenn man
a) Propanol-Derivate der Formel 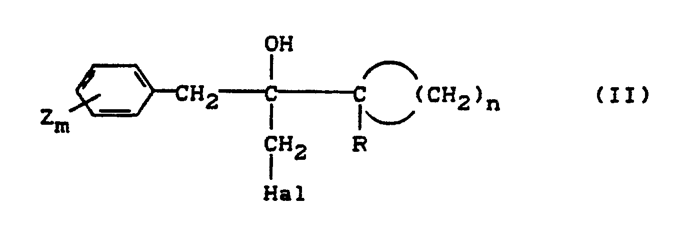 in welcher
   R, Z, m und n die oben angegebenen Bedeutungen haben und
   Hal für Chlor, Brom oder Iod steht,
   mit 1,2,4-Triazol der Formel 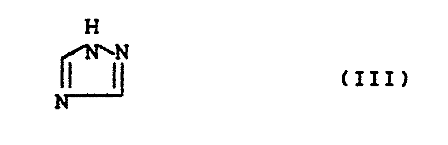 in Gegenwart eines Säurebindemittels und in Gegenwart eines Verdünnungsmittels umsetzt,
   oder
b) Oxirane der Formel 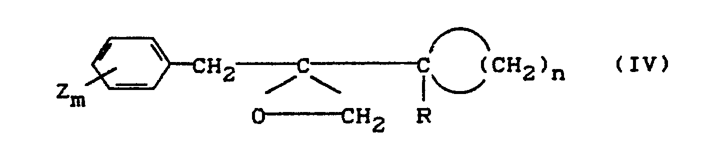 in welcher
   R, Z, m und n die oben angegebenen Bedeutungen haben,
   mit 1,2,4-Triazol der Formel 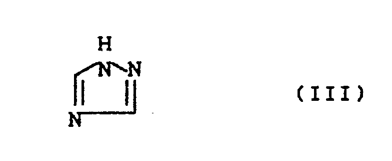 in Gegenwart eines Säurebindemittels und in Gegenwart eines Verdünnungsmittels umsetzt,
   oder
c) Triazolylmethyl-ketone der Formel 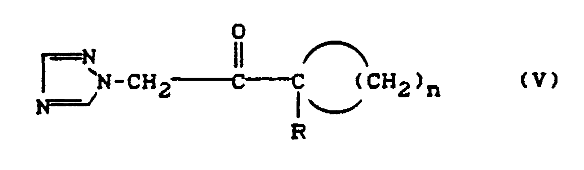 in welcher
   R und n die oben angegebenen Bedeutungen haben,
   mit metallorganischen Verbindungen der Formel 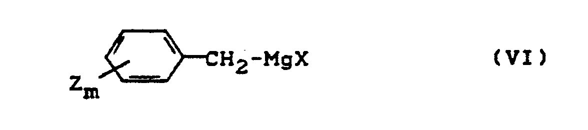 in welcher
   - Z und m: die oben angegebenen Bedeutungen haben
   und
   - X: für Chlor, Brom oder Jod steht,
   in Gegenwart eines Verdünnungsmittels umsetzt,

und gegebenenfalls anschließend en die so erhaltenen Verbindungen der Formel (I) eine Säure oder ein Metallsalz addiert.

Schließlich wurde gefunden, daß die neuen 1,2,4-Triazolyl-propanol-Derivate der Formel (I) sowie deren Säureadditions-Salze und Metallsalz-Komplexe starke fungizide Eigenschaften besitzen.

Die erfindungsgemäßen Stoffe enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemische.

Überraschenderweise besitzen die erfindungsgemäßen Stoffe bessere fungizide Eigenschaften als die konstitutionell ähnlichsten vorbekannten Verbindungen gleicher Wirkungsrichtung.

Wenn m in der Formel (I) für die Zahlen 2 oder 3 steht, können die für Z stehenden Reste gleich oder verschieden sein.

Zu den Säuren, die an die 1,2,4-Triazolyl-propanol-Derivate der Formel (I) addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure, und außerdem auch Saccharin.

Zu den Metallsalzen, die an die 1,2,4-Triazolyl-propanol-Derivate der Formel (I) addiert werden können, gehören vorzugsweise Salze von Metallen der II. bis IV. Haupt- und der I. und II. sowie IV. bis VIII. Nebengruppe des Periodensystems der Elemente.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für 1,2,4-Triazolyl-propanol-Derivate der Formel (I) seien die in der folgenden Tabelle aufgeführten Stoffe genannt.

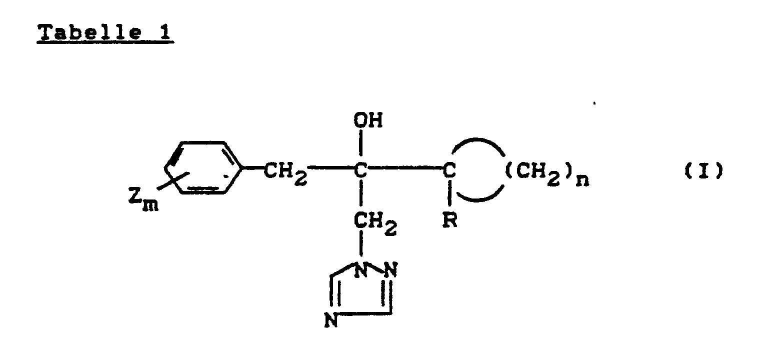

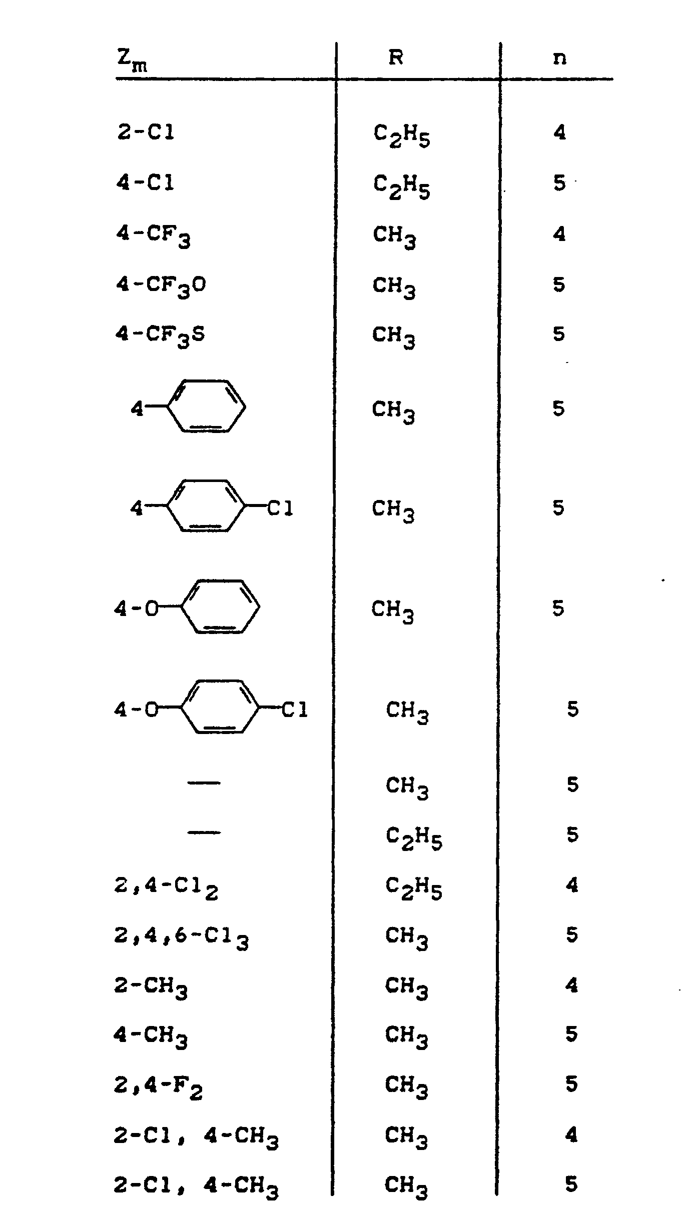

Verwendet man 1-Chlor-3-(2-chlorphenyl)-2-(1-methylcyclohex-1-yl)-propan-2-ol und 1,2,4-Triazol als Ausgangsstoffe, so kann der Verlauf des erfindungsgemäßen Verfahrens (a) durch das folgende Formelschema veranschaulicht werden:

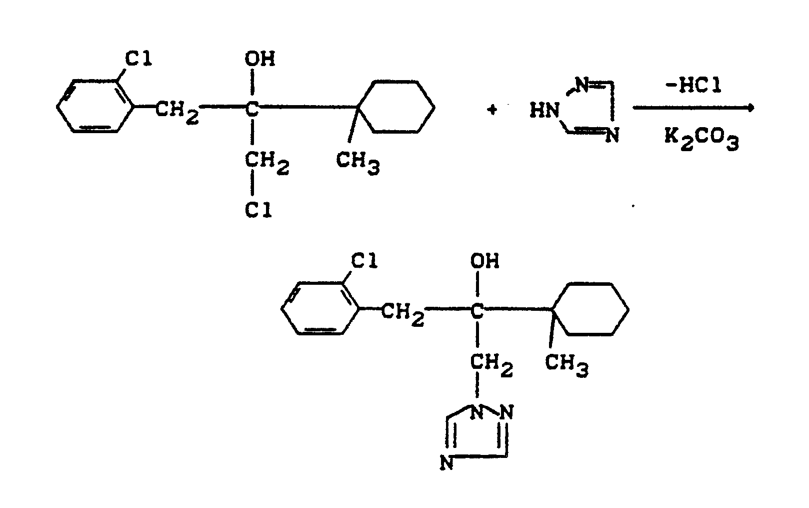

Verwendet man 2-[(2-Chlor-phenyl)-methyl]-2-(1-methylcyclopent-1-yl)-oxiran und 1,2,4-Triazol als Ausgangsstoffe, so kann der Verlauf des erfindungsgemäßen Verfahrens (b) durch das folgende Formelschema veranschaulicht werden:

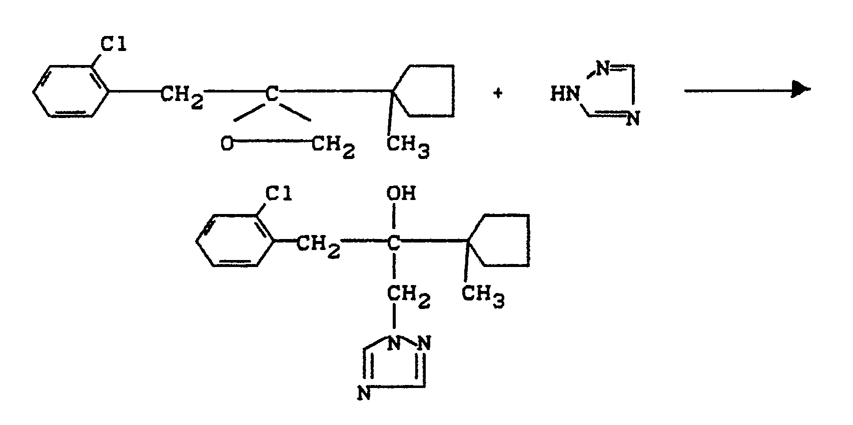

Verwendet man (1,2,4-Triazol-1-yl-methyl)-(1-methylcyclohex-1-yl)-keton und 4-Fluor-benzyl-magnesiumbromid als Ausgangsstoffe, so kann der Verlauf des erfindungsgemäßen Verfahrens (c) durch das folgende Formelschema veranschaulicht werden:

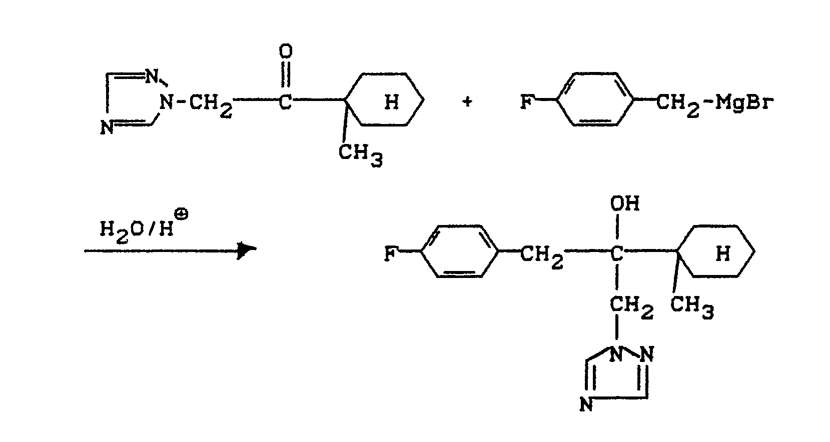

Die bei dem erfindungsgemäßen Verfahren (a) als Ausgangsstoffe benötigten Propanol-Derivate sind durch die Formel (II) definiert. In dieser Formel haben R, Z, m und n vorzugsweise diejenigen Bedeutungen, die bereits im Zusammenhang mit der Beschreibung der erfindungsgemäßen Stoffe der Formel (I) für diese Reste bzw. diese Indices genannt wurden. Hal steht vorzugsweise für Chlor oder Brom.

Die Propanol-Derivate der Formel (II) sind bisher noch nicht bekannt. Sie lassen sich herstellen, indem man Cycloalkylketone der Formel 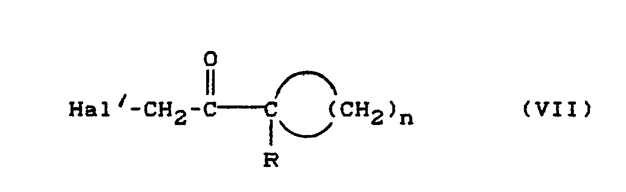
in welcher
R und n die oben angegebenen Bedeutungen haben und
Hal' für Chlor oder Brom steht,
mit metallorganischen Verbindungen der Formel 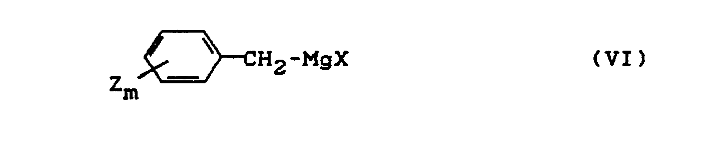 in welcher
X, Z und m die oben angegebene Bedeutung haben
in Gegenwart eines Verdünnungsmittels umsetzt.

Die bei der Herstellung der Propanol-Derivate nach dem obigen Verfahren als Ausgangsstoffe benötigten Cycloalkylketone der Formel (VII) sind teilweise bekannt, (vgl. EP-OS 0 055 427). Sie lassen sich herstellen, indem man Cycloalkyl-Verbindungen der Formel 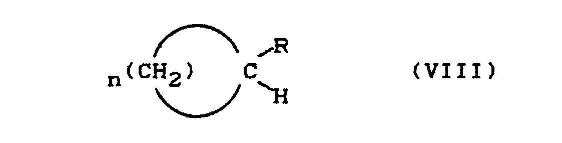
in welcher
R und n die oben angegebenen Bedeutungen haben,
mit 1,1-Dichlorethen der Formel

CH₂=CCl₂ (IX)

in Gegenwart von tert.-Butylchlorid und Aluminiumchlorid bei Temperaturen zwischen -20° C und +10° C umsetzt und die dabei entstehenden 2,2-Dichlorethenyl-cycloalkyl-Derivate der Formel 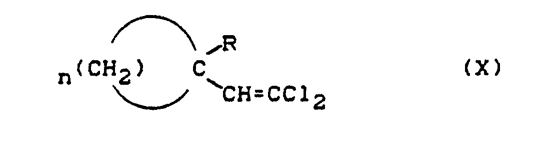
in welcher
R und n die oben angegebenen Bedeutungen haben,
mit Natriumphenolat in Gegenwart eines Säurebindemittels, wie z.B. Kaliumcarbonat, und in Gegenwart eines Verdünnungsmittels, wie z.B. N-Methylpyrrolidon, bei Temperaturen zwischen 50° C und 220° C umsetzt und die dabei entstehenden Stoffe der Formel 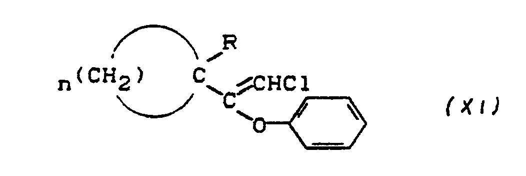
in welcher
R und n die oben angegebenen Bedeutungen haben,
mit Säuren, wie z.B. Ameisensäure, gegebenenfalls in Gegenwart eines Verdünnungsmittels, wie z.B. Methanol oder Ethanol, und in Gegenwart von Wasser umsetzt.

Die bei dem obigen Verfahren zur Herstellung von Propanol-Derivaten der Formel (II) als Reaktionskomponenten benötigten metallorganischen Verbindungen der Formel (VI) sind bekannt oder lassen sich nach prinzipiell bekannten Methoden synthetisieren. So erhält man Verbindungen der Formel (VI), indem man Benzylhalogenide der Formel 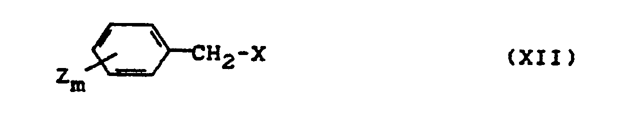
in welcher
X, Z und m die oben angegebene Bedeutung haben,
mit Magnesium in Gegenwart eines inerten Verdünnungsmittels, wie z.B. Diethylether, bei Temperaturen zwischen 0° C und 50° C umsetzt.

Die Benzylhalogenide der Formel (XII) sind allgemein bekannte Verbindungen der organischen Chemie.

Als Verdünnungsmittel kommen bei dem obigen Verfahren zur Herstellung von Propanol-Derivaten der Formel (II) alle für derartige Umsetzungen üblichen inerten organischen Solventien in Betracht. Vorzugsweise verwendbar sind Ether, wie Diethylether, Tetrahydrofuran und Dioxan.

Die Reaktionstemperaturen können bei der Durchführung des obigen Verfahrens zur Herstellung von Propanol-Derivaten der Formel (II) innerhalb eines bestimmten Bereiches variiert werden. Im allgemeinen arbeitet man bei Temperaturen zwischen -80° C und +100° C, vorzugsweise zwischen -80° C und+60° C.

Bei der Durchführung des obigen Verfahrens zur Herstellung von Propanol-Derivaten der Formel (II) arbeitet man im allgemeinen unter Normaldruck. Es ist jedoch auch möglich, unter erhöhtem oder vermindertem Druck zu arbeiten.

Bei der Durchführung des obigen Verfahrens zur Herstellung von Propanol-Derivaten der Formel (II) setzt man auf 1 Mol an Cycloalkylketon der Formel (VII) im allgemeinen 1 bis 1,2 Mol an metallorganischer Verbindung der Formel (VI) ein, die zweckmäßigerweise unmittelbar zuvor hergestellt und in situ weiterverarbeitet wird. Die Aufarbeitung erfolgt nach üblichen Methoden. Im allgemeinen geht man so vor, daß man zunächst ansäuert und mit Wasser versetzt, dann die organische Phase abtrennt, wäscht und nach dem Trocknen einengt.

Als Säurebindemittel kommen bei der Durchführung des erfindungsgemäßen Verfahrens (a) alle üblichen anorganischen und organischen Basen infrage. Vorzugsweise verwendbar sind Alkalicarbonate, wie Natrium- und Kaliumcarbonat, ferner Alkalimetallhydroxide, wie Natrium- und Kaliumhydroxid, außerdem Alkalimetallalkoholate, wie Natrium- und Kaliummethylat und -ethylat sowie Kaliumtert.-butylat, und weiterhin niedere tertiäre Alkylamine, Cycloalkylamine und Aralkylamine, wie insbesondere Triethylamin.

Als Verdünnungsmittel kommen bei der Durchführung des erfindungsgemäßen Verfahrens (a) alle üblichen inerten organischen Solventien in Betracht. Vorzugsweise verwendbar sind Nitrile, wie Acetonitril, ferner aromatische Kohlenwasserstoffe, wie Benzol, Toluol und Dichlorbenzol, außerdem Formamide, wie Dimethylformamid, sowie stark polare Lösungsmittel, wie Dimethylsulfoxid und Hexamethylphosphorsäuretriamid.

Die Reaktionstemperaturen können bei der Durchführung des erfindungsgemäßen Verfahrens (a) innerhalb eines größeren Bereiches variiert werden. Im allgemeinen arbeitet man bei Temperaturen zwischen 0° C und 200° C, vorzugsweise zwischen 60° C und 150° C.

Das erfindungsgemäße Verfahren (a) wird ebenso wie die erfindungsgemäßen Verfahren (b) und (c) im allgemeinen unter Normaldruck durchgeführt. Es ist jedoch jeweils auch möglich, unter erhöhtem oder vermindertem Druck zu arbeiten.

Bei der Durchführung des erfindungsgemäßen Verfahrens (a) setzt man auf 1 Mol an Propanol-Derivat der Formel (II) im allgemeinen 1 bis 4 Mol 1,2,4-Triazol der Formel (III) sowie 1 bis 3 Mol an Säurebindemittel ein. In manchen Fällen ist es zweckmäßig, unter Schutzgasatmosphäre zu arbeiten. Die Aufarbeitung erfolgt nach üblichen Methoden. Im allgemeinen geht man so vor, daß man das Reaktionsgemisch durch Abziehen des Verdünnungsmittels einengt, den verbleibenden Rückstand in einem mit Wasser wenig mischbaren organischen Lösungsmittel aufnimmt, die organische Phase wäscht und nach dem Trocknen einengt. Das verbleibende Produkt kann gegebenenfalls weiteren Reinigungsverfahren unterzogen werden.

Die bei dem erfindungsgemäßen Verfahren (b) als Ausgangsstoffe benötigten Oxirane der Formel (IV) sind bisher noch nicht bekannt. Sie lassen sich herstellen, indem man Propanol-Derivate der Formel 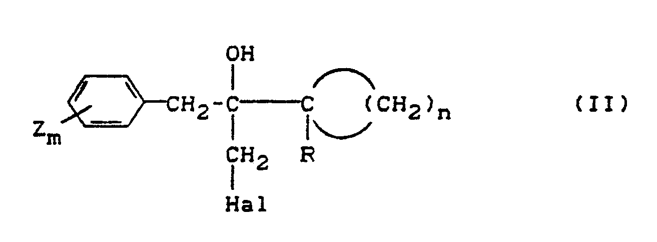
in welcher
R, Z, Hal, m und n die oben angegebenen Bedeutungen haben,
mit Basen in Gegenwart eines Verdünnungsmittels umsetzt.

Als Basen kommen bei der Herstellung von Oxiranen der Formel (IV) nach dem obigen Verfahren alle üblicherweise für derartige Umsetzungen geeigneten anorganischen und organischen Basen in Betracht. Vorzugsweise verwendbar sind alle diejenigen Basen, die bereits im Zusammenhang mit der Beschreibung des erfindungsgemäßen Verfahrens (a) als bevorzugte Säurebindemittel genannt wurden.

Die Reaktionstemperaturen können bei der Herstellung von Oxiranen nach dem obigen Verfahren innerhalb eines bestimmten Bereiches variiert werden. Im allgemeinen arbeitet man bei Temperaturen zwischen -30° C und +100° C, vorzugsweise zwischen -20° C und +60° C.

Bei der Durchführung des obigen Verfahrens zur Herstellung von Oxiranen der Formel (IV) arbeitet man im allgemeinen unter Normaldruck. Es ist jedoch auch möglich, unter erhöhtem oder vermindertem Druck zu arbeiten.

Bei der Durchführung des obigen Verfahrens zur Herstellung von Oxiranen der Formel (IV) setzt man im allgemeinen auf 1 Mol an Propanol-Derivat der Formel (II) 1 bis 3 Mol an Base ein. Die Aufarbeitung erfolgt nach üblichen Methoden.

Als Säurebindemittel und als Verdünnungsmittel kommen bei der Durchführung des erfindungsgemäßen Verfahrens (b) alle üblicherweise für derartige Umsetzungen einsetzbaren Säurebindemittel und Verdünnungsmittel infrage. Vorzugsweise verwendbar sind alle diejenigen Säurebindemittel und Verdünnungsmittel, die bereits im Zusammenhang mit der Beschreibung des erfindungsgemäßen Verfahrens (a) als bevorzugte Säurebindemittel und Verdünnungsmittel genannt wurden.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen 0° C und 200° C, vorzugsweise zwischen 60° C und 150° C.

Bei der Durchführung des erfindungsgemäßen Verfahrens (b) setzt man auf 1 Mol an Oxiran der Formel (IV) im allgemeinen 1 bis 2 Mol an 1,2,4-Triazol der Formel (III) und 1 bis 2 Mol an Säurebindemittel ein. Die Aufarbeitung erfolgt nach üblichen Methoden.

Die bei dem erfindungsgemäßen Verfahren (c) als Ausgangsstoffe benötigten Triazolylmethyl-ketone der Formel (V) sind teilweise bekannt. Sie lassen sich herstellen, indem man Cycloalkylketone der Formel 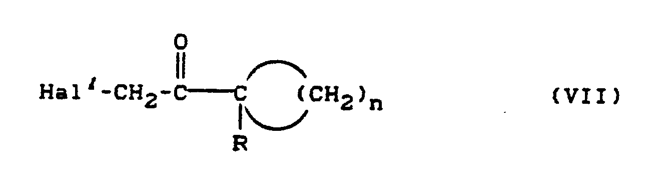
in welcher
R, Hal' und n die oben angegebenen Bedeutungen haben,
mit 1,2,4-Triazol der Formel 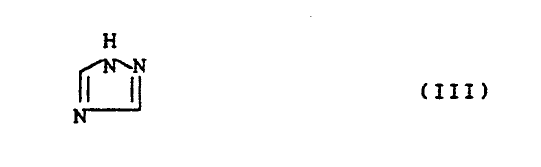
in Gegenwart eines Säurebindemittels und gegebenenfalls in Gegenwart eines Verdünnungsmittels umsetzt.

Als Säurebindemittel und als Verdünnungsmittel kommen bei der Herstellung von Triazolylmethyl-ketonen der Formel (V) nach dem obigen Verfahren alle üblicherweise für derartige Umsetzungen verwendbaren Säurebindemittel und Verdünnungsmittel in Frage. Vorzugsweise verwendbar sind alle diejenigen Säurebindemittel, die bereits im Zusammenhang mit der Beschreibung des erfindungsgemäßen Verfahrens (a) als bevorzugte Säurebindemittel genannt wurden. Als Verdünnungsmittel kommen vorzugsweise Ketone, wie Aceton, und Nitrile, wie Acetonitril, in Betracht.

Die Reaktionstemperaturen können bei der Herstellung von Triazolylmethyl-ketonen der Formel (V) nach dem obigen Verfahren innerhalb eines größeren Bereiches variiert werden. Im allgemeinen arbeitet man bei Temperaturen zwischen 0° C und 120° C, vorzugsweise zwischen 20° C und 100° C.

Das obige Verfahren zur Herstellung von Triazolylmethylketonen der Formel (V) wird im allgemeinen unter Normaldruck durchgeführt.

Bei der Durchführung des obigen Verfahrens zur Herstellung von Triazolylmethyl-ketonen der Formel (V) setzt man auf 1 Mol an Cycloalkylketon der Formel (VII) im allgemeinen auf 1 bis 4 Mol an 1,2,4-Triazol der Formel (III) sowie 1 bis 3 Mol an Säurebindemittel ein. Die Aufarbeitung erfolgt nach üblichen Methoden.

Die bei dem erfindungsgemäßen Verfahren (c) als Reaktionskomponenten benötigten metallorganischen Verbindungen der Formel (VI) wurden bereits im Zusammenhang mit der Beschreibung des Verfahrens zur Herstellung der Propanol-Derivate der Formel (II) erwähnt.

Bei der Durchführung des erfindungsgemäßen Verfahrens (c) kommen als Verdünnungsmittel alle üblichen inerten organischen Solventien in Betracht. Vorzugsweise verwendbar sind Ether, wie Diethylether, Tetrahydrofuran und Dioxan.

Die Reaktionstemperaturen können auch bei der Durchführung des erfindungsgemäßen Verfahrens (c) in einem größeren Bereich variiert werden. Im allgemeinen arbeitet man bei Temperaturen zwischen -80° C und +60° C, vorzugsweise zwischen -70° C und +50° C.

Bei der Durchführung des erfindungsgemäßen Verfahrens (c) setzt man auf 1 Mol an Triazolylmethyl-keton der Formel (V) im allgemeinen 0,8 bis 2 Mol an metallorganischer Verbindung der Formel (VI) ein, die zweckmäßigerweise unmittelbar zuvor hergestellt und in situ weiterverarbeitet wird. Die Aufarbeitung erfolgt nach üblichen Methoden.

Die nach den erfindungsgemäßen Verfahren erhältlichen 1,2,4-Triazolyl-propanol-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreide- und Reiskrankheiten, wie Pseudocercosporella herpotrichoides, Septoria nodorum, Cochliobolus sativus, Pyrenophora teres, Fusarium spp., Erysiphe, Pyricularia und Pellicularia. Sie lassen sich mit besonders gutem Erfolg gegen Pyricularia oryzae und Pellicularia sasakii an Reis verwenden. Ferner eignen sie sich zur Bekämpfung von Venturia und Botrytis im Obst-, Wein- und Gemüsebau. Außerdem zeigen die erfindungsgemäßen Stoffe auch eine gute in-vitro-Wirkung.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Wirkstoffe gehen aus den folgenden Beispielen hervor.

### Herstellungsbeispiele

### Beispiel 1

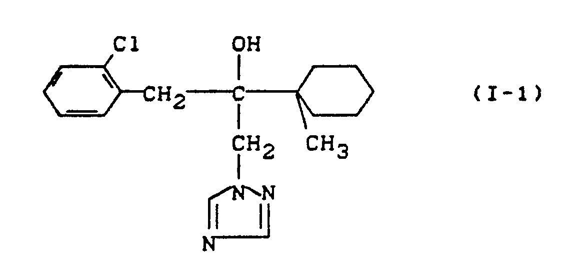

Unter Stickstoffatomosphäre werden 12 g (174 mMol) 1,2,4-Triazol und 17 g (123 mMol) Kaliumcarbonat in 30 ml absolutem Dimethylformamid vorgelegt und auf 80° C erwärmt. Bei dieser Temperatur tropft man unter Rühren eine Lösung von 16,7 g (55,5 mMol) 1-Chlor-3-(2-chlorphenyl)-2-(1-methyl-cyclohex-1-yl)-propan-2-ol in 20 ml absolutem Dimethylformamid hinzu. Danach wird das Reaktionsgemisch noch 8 Stunden bei 80° C gerührt. Der Niederschlag wird abgesaugt, und das Filtrat wird durch Abziehen des Verdünnungsmittels unter vermindertem Druck eingeengt. Man nimmt den Rückstand in Essigsäureethylester auf, wäscht mit Wasser und zieht nach dem Trocknen über Natriumsulfat das Lösungsmittel unter vermindertem Druck ab. Das verbleibende Produkt wird über eine Kieselgel-Säule mit Dichlormethan/Ethanol = 99:1 als Laufmittel chromatographiert. Man erhält auf diese Weise 8,6 g (46,5 % der Theorie) an 1-(2-Chlorphenyl)-2-(1-methyl-cyclohex-1-yl)-3-(1,2,4-triazol-1-yl)-propan-2-ol in Form einer Festsubstanz vom Schmelzpunkt 105° C.

### Herstellung des Vorproduktes der Formel

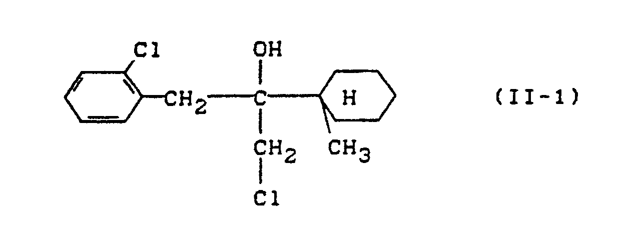

Unter Stickstoffatmosphäre wird eine Lösung von 10,1 g (63 mMol) 2-Chlor-benzylchlorid in 30 ml absolutem Diethylether so in ein Gemisch von 1,6 g (66 mMol) Magnesium-Spänen und einem Iod-Kristall in 10 ml absolutem Diethylether eingetropft, daß der Diethylether leicht siedet. Man rührt 30 Minuten bei Siedetemperatur nach, kühlt dann auf Raumtemperatur ab und tropft die so hergestellte Grignard-Lösung unter Rühren in eine Lösung von 10 g (57 mMol) 1-Chloracetyl-1-methyl-cyclohexan in 40 ml absolutem Diethylether. Das Reaktionsgemisch wird 4 Stunden bei 20° C gerührt, dann mit gesättigter, wäßriger Ammoniumchlorid-Lösung versetzt und auf Wasser gegossen. Man extrahiert mit Essigsäureethylester, wäscht die vereinigten organischen Phasen mit Wasser, trocknet über Natriumsulfat und engt durch Abziehen des Lösungsmittels unter vermindertem Druck ein. Es verbleiben 16,7 g (97 % der Theorie) 1-Chlor-3-(2-chlorphenyl)-2-(1-methylcyclohex-1-yl)-propan-2-ol in Form eines Öls.
¹H-NMR (200 MHz, CDCl₃):
δ = 1,13 (s) und 1,05-1,7 (m, 13H), 1,82 (d, 1H), 2,62 (dd, 1H), 3,06 (d, 1H), 3,38 (d, 1H), 7,1-7,38 (m, 4H) ppm.

### Beispiel 2

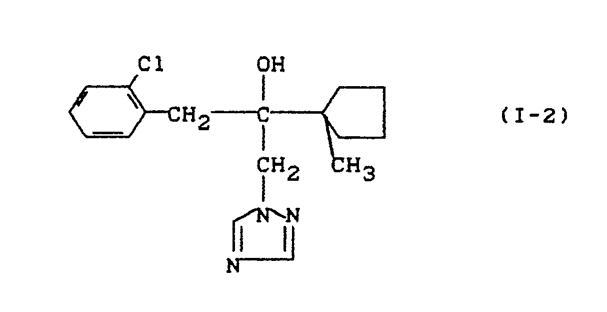

Unter Stickstoffatmosphäre werden 13 g (188 mMol) 1,2,4-Triazol und 18 g (130 mMol) Kaliumcarbonat in 30 ml absolutem Dimethylformamid vorgelegt und auf 80° C erwärmt. Bei dieser Temperatur tropft man unter Rühren eine Lösung von 17,1 g (59,6 mMol) 1-Chlor-3-(2-chlorphenyl)-2-(1-methyl-cyclopent-1-yl)-propan-2-ol in 20 ml absolutem Dimethylformamid hinzu. Danach wird das Reaktionsgemisch noch 8 Stunden bei 80° C gerührt. Der Niederschlag wird abgesaugt, und das Filtrat wird durch Abziehen des Verdünnungsmittels unter vermindertem Druck eingeengt. Man nimmt den Rückstand in Essigsäureethylester auf, wäscht mit Wasser und zieht nach dem Trocknen über Natriumsulfat das Lösungsmittel unter vermindertem Druck ab. Das verbleibende Produkt wird über eine Kieselgel-Säule mit Dichlormethan/Ethanol = 99:1 als Laufmittel chromatographiert. Man erhält auf diese Weise 6,8 g (35,7 % der Theorie) an 1-(2-Chlorphenyl)-2-(1-methyl-cyclopent-1-yl)-3-(1,2,4-triazol-1-yl)-propan-2-ol in Form einer Festsubstanz vom Schmelzpunkt 110° C.

### Herstellung von Ausgangssubstanzen:

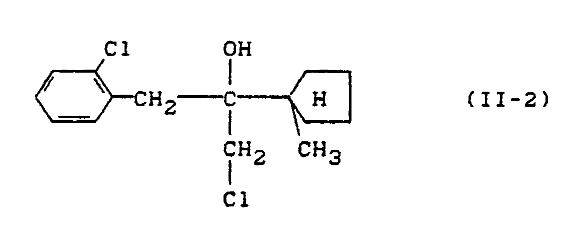

Unter Stickstoffatmosphäre wird eine Lösung von 11 g (68 mMol) 2-Chlor-benzylchlorid in 30 ml absolutem Diethylether so in ein Gemisch von 1,7 g (70 mMol) Magnesium-Spänen und einem Iod-Kristall in 10 ml absolutem Diethylether eingetropft, daß der Diethylether leicht siedet. Man rührt 30 Minuten bei Siedetemperatur nach, kühlt dann auf Raumtemperatur ab und tropft die so hergestellte Grignard-Lösung unter Rühren in eine Lösung von 10 g (62 mMol) 1-Chloracetyl-1-methyl-cyclopentan in 40 ml absolutem Diethylether. Das Reaktionsgemisch wird 4 Stunden bei 20° C gerührt, dann mit gesättigter, wäßriger Ammoniumchlorid-Lösung versetzt und auf Wasser gegossen. Man extrahiert mit Essigsäureethylester, wäscht und engt durch Abziehen des Lösungsmittels unter vermindertem Druck ein. Es verbleiben 17,1 g (96 % der Theorie) 1-Chlor-3-(2-chlorphenyl)-2-(1-methylcyclopent-1-yl)-propan-2-ol in Form eines Öls.
¹H-NMR (200 MHz, CDCl₃):
δ = 1,16 (s, 3H), 1,3-1,8 (m, 8H), 1,9 (d, 1H), 2,45 (dd, 1H), 3,06 (d, 1H), 3,42 (d, 1H), 7,1-7,27 (m, 4H) ppm.

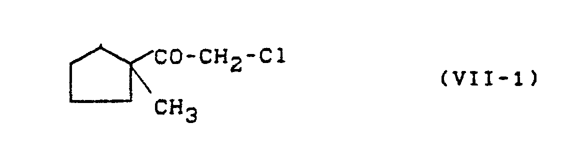

Ein Gemisch aus 1040 g (4,4 Mol) 2-Chlor-1-(1-methylcyclopent-1-yl)-1-phenoxy-ethen, 4 Litern Ameisensäure und 400 ml Wasser wird 1 Stunde unter Rühren auf 80° C erhitzt. Nach dem Abkühlen auf Raumtemperatur wird das Reaktionsgemisch mit Methylenchlorid verdünnt, danach zweimal mit Wasser und dreimal mit verdünnter, wäßriger Natronlauge ausgeschüttelt. Die organische Phase wird nach dem Trocknen über Natriumsulfat durch Abziehen des Lösungsmittels unter vermindertem Druck eingeengt. Der verbleibende Rückstand wird einer fraktionierten Vakuumdestillation unterworfen.

Man erhält auf diese Weise 604 g (85,5 % der Theorie) an 1-Chloracetyl-1-methyl-cyclopentan in Form einer Flüssigkeit vom Siedepunkt 85-95° C/10 mbar.
NMR (CDCl₃): δ = 1,3 (s, 3H), 1,4-2,2 (m, 8H), 4,4 (s, 2H) ppm.

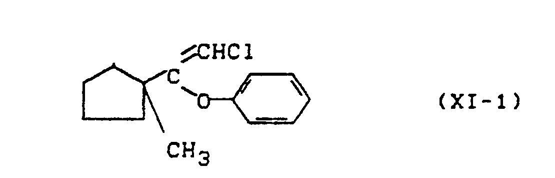

Ein Gemisch aus 1074 g (6 Mol) 1-(2,2-Dichlorethenyl)-1-methyl-cyclopentan, 835 g (7,2 Mol) Natriumphenolat, 828 g (6 Mol) wasserfreiem Kaliumcarbonat und 6 Litern N-Methylpyrrolidon wird 5 Stunden auf 200° C erhitzt. Nach dem Abkühlen des Reaktionsgemisches auf Raumtemperatur verdünnt man zunächst mit Methylenchlorid und schüttelt dann einmal mit Wasser und mehrfach mit verdünnter, wäßriger Natronlauge aus. Die organische Phase wird über Natriumsulfat getrocknet und durch Abziehen des Lösungsmittels unter vermindertem Druck eingeengt. Es verbleiben 2,5 kg eines Rückstandes, der über eine Kolonne fraktioniert destilliert wird. Durch Auffangen der Fraktion, die bei 0,1 mbar zwischen 100° C und 120 ° C siedet, erhält man 1043 g eines Produktes, das gemäß Gaschromatogramm zu 83,1 % aus 2-Chlor-1-(1-methylcyclopent-1-yl)-1-phenoxy-ethen besteht. Die Ausbeute errechnet sich danach zu 73,5 %.

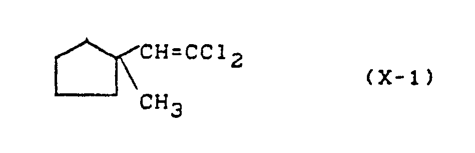

In ein Gemisch aus 575 g (6,85 Mol) Methyl-cyclopentan, 634 g (6,85 Mol) tert.-Butylchlorid und 1,99 kg (20,55 Mol) 1,1-Dichlorethen werden bei 0° C unter Rühren und weiterer Kühlung 150 g (1,13 Mol) gepulvertes Aluminiumchlorid portionsweise gegeben. Nach beendeter Zugabe wird zunächst 2 Stunden nachgerührt und dann noch einmal mit 50 g Aluminiumchlorid versetzt. Das Reaktionsgemisch wird 2 Stunden bei 0° C bis 10° C nachgerührt und dann auf Eis und verdünnte Salzsäure gegossen. Man extrahiert mehrfach mit Methylenchlorid, trocknet die vereinigten organischen Phasen über Natriumsulfat und engt durch Abziehen des Lösungsmittels unter vermindertem Druck ein. Es verbleiben 1,4 kg eines Produktes, das einer fraktionierten Destillation unter vermindertem Druck unterworfen wird. Dabei werden zwei Fraktionen aufgefangen, und zwar
a) 434 g eines Produktes, das bei 30 mbar zwischen 27° C und 66° C siedet, und
b) 622 g eines Produktes, das bei 25 mbar zwischen 90° C und 130° C siedet.

Die zweite Fraktion besteht gemäß Gaschromatogramm zu 85 % aus 1-(2,2-Dichlorethenyl)-1-methyl-cyclopentan. Dieses Produkt wird bei den weiteren Umsetzungen verwendet.

Nach den zuvor genannten Methoden werden auch die in der nachfolgenden Tabelle 2 aufgeführten erfindungsgemäßen Stoffe hergestellt.

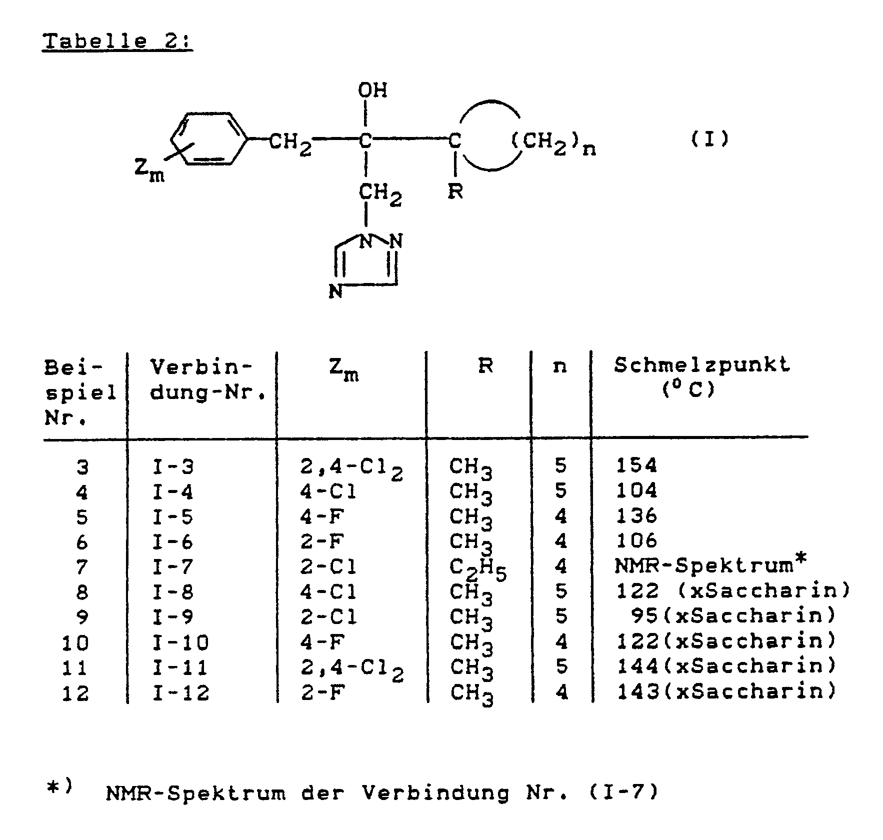

¹H-NMR (200 MHz, CDCl₃):
δ = 1,03 (t, 3H), 1,35-2,05 (m, 10H), 2,95 (d, 1H), 3,42 (d, 1H), 4,20 (d, 1H), 4,21 (s, 1H), 4,35 (d, 1H), 7,05-7,42 (m, 4H), 7,63 (s, 1H), 7,72 (s, 1H) ppm.

### Beispiel A

### Pyricularia-Test (Reis) /protektiv

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25° C aufgestellt.

4 Tage nach der Inokulation erfolgt die Auswertung des Krankheitsbefalls.

In diesem Test zeigen die erfindungsgemäßen Stoffe (I-1) und (I-2) eine sehr gute Wirksamkeit.

### Beispiel B

### Pellicularia-Test (Reis) / protektiv

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Mengen Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25° C und 100 % relativer Luftfeuchtigkeit aufgestellt.

5 bis 8 Tage nach der Inokulation erfolgt die Auswertung des Krankheitsbefalls.

In diesem Test zeigen die erfindungsgemäßen Stoffe (I-1) (I-2) eine sehr gute Wirksamkeit.

### Beispiel C

### Botrytis-Test (Paprika) / protektiv

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension von Botrytis cinerea inokuliert.

Die Pflanzen verbleiben dann 4 Tage bis zur Auswertung bei 20° C und 100 % relativer Luftfeuchtigkeit in einem Inkubationsschrank.

In diesem Test zeigen die erfindungsgemäßen Stoffe (I-1) und (I-2) eine sehr gute Wirksamkeit.

### Beispiel D

### Venturia-Test (Apfel) / protektiv

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20° C und 100 % relativer Luftfeuchtigkeit in einer Inkubationskabine.

Die Pflanzen werden dann im Gewächshaus bei 20° C und einer relativen Luftfeuchtigkeit von ca. 70 % aufgestellt.

12 Tage nach der Inokulation erfolgt die Auswertung.

In diesem Test zeigen die erfindungsgemäßen Stoffe (I-1) und (I-2) eine sehr gute Wirksamkeit.

### Beispiel E

### Erysiphe-Test (Gerste) / protektiv

- Lösungsmittel:: 100 Gewichsteile Dimethylformamid
- Emulgator:: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Stoffe (I-1) und (I-2) eine sehr gute Wirksamkeit.

### Beispiel F

### Botrytis-Test (Buschbohne)/protektiv

- Lösungsmittel:: 4,7 Gewichtsteile Aceton
- Emulgator:: 0,3 Gewichtsteile Alkyl-Aryl-Polyklykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20° C aufgestellt. 3 Tage nach der Inokulation wird die Größe der Befallsflecken auf den Blättern ausgewertet.

In diesem Test zeigen die erfindungsgemäßen Stoffe (I-1) und (I-2) eine sehr gute Wirksamkeit.

### Beispiel G

### Venturia-Test (Apfel) / kurativ

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Konidiensuspension des Apfelschorferregers (Venturia inaequalis) inokuliert. Die Pflanzen verbleiben 1 Tag bei 20° C und 100 % relativer Luftfeuchtigkeit in einer Inkubationskabine und werden dann im Gewächshaus aufgestellt. Nach einer angegebenen Stundenzahl werden die Pflanzen mit der Wirkstoffzubereitung tropfnaß gespritzt.

Die Pflanzen werden dann im Gewächshaus bei 20° C und einer relativen Luftfeuchtigkeit von ca. 70 % aufgestellt.

12 Tage nach der Inokulation erfolgt die Auswertung.

In diesem Test zeigen die erfindungsgemäßen Stoffe (I-1) und (I-2) eine sehr gute Wirksamkeit.

## Patentansprüche

1. 1,2,4-Triazolyl-propanol-Derivate der Formel 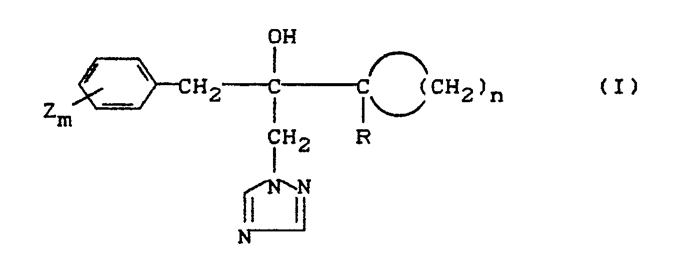 in welcher
R für Methyl oder Ethyl steht,
Z für Fluor, Chlor, Trifluormethyl, Trifluormethoxy, Trifluormethylthio, Methyl, gegebenenfalls durch Fluor oder Chlor substituiertes Phenyl oder gegebenenfalls durch Fluor oder Chlor substituiertes Phenoxy steht,
m für 0, 1, 2 oder 3 steht und
n für 4 oder 5 steht,
sowie deren Säureadditions-Salze und Metallsalz-Komplexe.

2. Verfahren zur Herstellung von 1,2,4-Triazolylpropanol-Derivaten der Formel 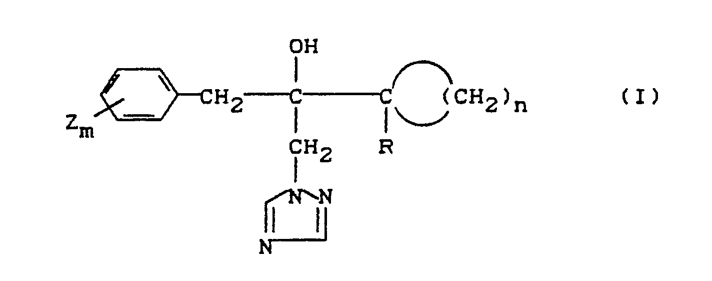 in welcher
R für Methyl oder Ethyl steht,
Z für Fluor, Chlor, Trifluormethyl, Trifluormethoxy, Trifluormethylthio, Methyl, gegebenenfalls durch Fluor oder Chlor substituiertes Phenyl oder gegebenenfalls durch Fluor oder Chlor substituiertes Phenoxy steht,
m für 0, 1, 2 oder 3 steht und
n für 4 oder 5 steht,
sowie von deren Säureadditions-Salzen und Metallsalz-Komplexen, dadurch gekennzeichnet, daß man
a) Propanol-Derivate der Formel 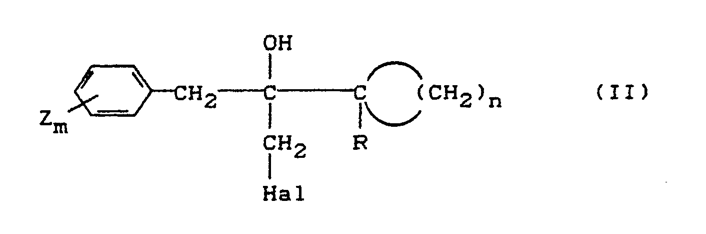 in welcher
R, Z, m und n die oben angegebenen Bedeutungen haben und
Hal für Chlor, Brom oder Iod steht,
mit 1,2,4-Triazol der Formel 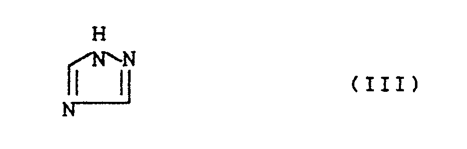 in Gegenwart eines Säurebindemittels und in Gegenwart eines Verdünnungsmittels umsetzt,
oder
b) Oxirane der Formel 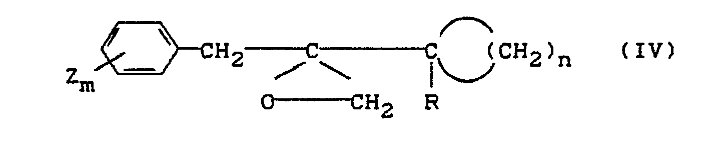 in welcher
R, Z, m und n die oben angegebenen Bedeutungen haben,
mit 1,2,4-Triazol der Formel 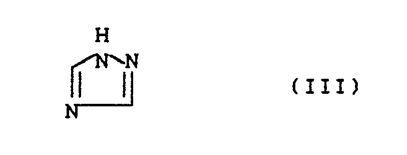 in Gegenwart eines Säurebindemittels und in Gegenwart eines Verdünnungsmittels umsetzt,
oder
c) Triazolylmethyl-ketone der Formel 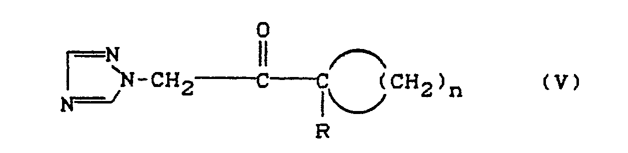
in welcher
R und n die oben angegebenen Bedeutungen haben,
mit metallorganischen Verbindungen der Formel 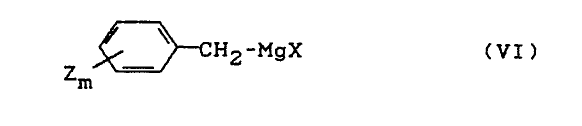 in welcher
Z und m die oben angegebenen Bedeutungen haben
und
X für Chlor, Brom oder Jod steht,
in Gegenwart eines Verdünnungsmittels umsetzt,
und gegebenenfalls anschließend an die so erhaltenen Verbindungen der Formel (I) eine Säure oder ein Metallsalz addiert.

3. Fungizide Mittel, gekennzeichnet durch einen Gehalt an mindestens einem 1,2,4-Triazolyl-propanol-Derivat der Formel (I) gemäß Anspruch 1 bzw. an einem Säureadditions-Salz oder Metallsalz-Komplex eines 1,2,4-Triazolyl-propanol-Derivates der Formel (I).

4. Verwendung von 1,2,4-Triazolyl-propanol-Derivaten der Formel (I) gemäß Anspruch 1 bzw. von deren Säureadditions-Salzen und Metallsalz-Komplexen zur Bekämpfung von Pilzen sowie zur Regulierung des Pflanzenwachstums.

5. Propanol-Derivate der Formel 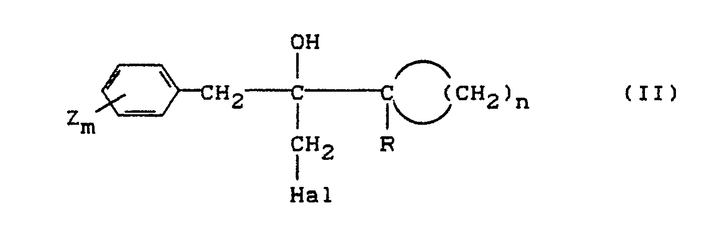 in welcher
R für Methyl oder Ethyl steht,
Z für Fluor, Chlor, Trifluormethyl, Trifluormethoxy, Trifluormethylthio, Methyl, gegebenenfalls durch Fluor oder Chlor substituiertes Phenyl oder gegebenenfalls durch Fluor oder Chlor substituiertes Phenoxy steht,
m für 0, 1, 2 oder 3 steht,
n für 4 oder 5 steht und
Hal für Chlor, Brom oder Iod steht.

6. Verfahren zur Herstellung von Propanol-Derivaten der Formel 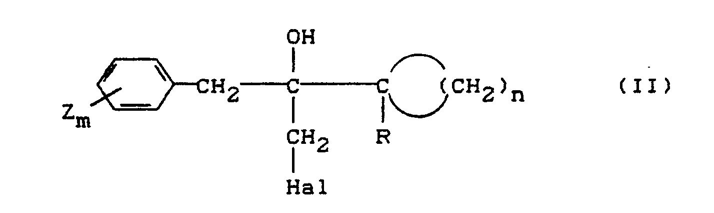 in welcher
R für Methyl oder Ethyl steht,
Z für Fluor, Chlor, Trifluormethyl, Trifluormethoxy, Trifluormethylthio, Methyl, gegebenenfalls durch Fluor oder Chlor substituiertes Phenyl oder gegebenenfalls durch Fluor oder Chlor substituiertes Phenoxy steht,
m für 0, 1, 2 oder 3 steht,
n für 4 oder 5 steht und
Hal für Chlor, Brom oder Iod steht,
dadurch gekennzeichnet, daß man Cycloalkylketone der Formel 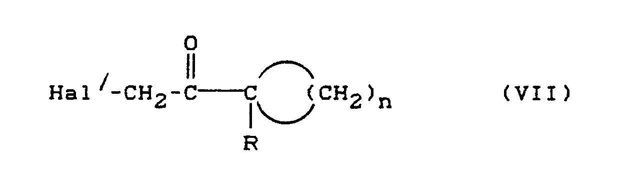 in welcher
R und n die oben angegebenen Bedeutungen haben und
Hal' für Chlor oder Brom steht,
mit metallorganischen Verbindungen der Formel 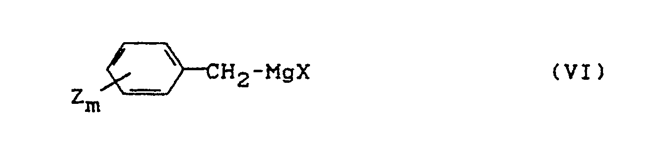 in welcher
X, Z und m die oben angegebene Bedeutung haben,
in Gegenwart eines Verdünnungsmittels umsetzt.

7. Oxirane der Formel 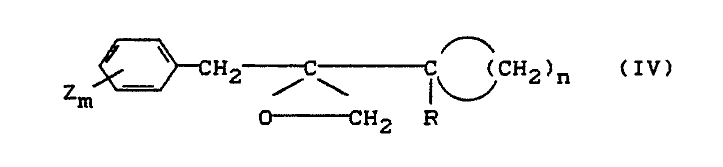 in welcher
R für Methyl oder Ethyl steht,
Z für Fluor, Chlor, Trifluormethyl, Trifluormethoxy, Trifluormethylthio, Methyl, gegebenenfalls durch Fluor oder Chlor substituiertes Phenyl oder gegebenenfalls durch Fluor oder Chlor substituiertes Phenoxy steht,
m für 0, 1, 2 oder 3 steht und
n für 4 oder 5 steht.

8. Verfahren zur Herstellung von Oxiranen der Formel 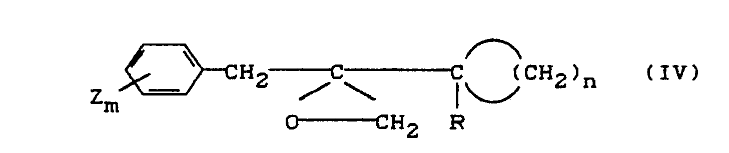 in welcher
R für Methyl oder Ethyl steht,
Z für Fluor, Chlor, Trifluormethyl, Trifluormethoxy, Trifluormethylthio, Methyl, gegebenenfalls durch Fluor oder Chlor substituiertes Phenyl oder gegebenenfalls durch Fluor oder Chlor substituiertes Phenoxy steht,
m für 0, 1, 2 oder 3 steht und
n für 4 oder 5 steht,
dadurch gekennzeichnet, daß man Propanol-Derivate der Formel 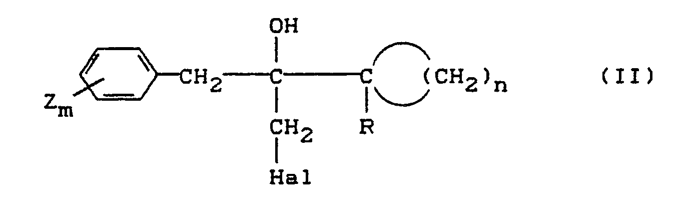 in welcher
R, Z, m und n die oben angegebenen Bedeutungen haben und
Hal für Chlor, Brom oder Iod steht,
mit Basen in Gegenwart eines Verdünnungsmittels umsetzt.
